# EUROPÄISCHE PATENTSCHRIFT

(11) **EP 1 576 973 B1**
(45) Veröffentlichungstag und Bekanntmachung des Hinweises auf die Patenterteilung: **27.03.2013**
(21) Anmeldenummer: 04007887.5
(22) Anmeldetag: 01.04.2004
(51) Int. Cl.: A61M 1/36

(54) **Bestrahlungsgerät für Blut**
Device for the irradiation of blood
Appareil pour l'irradiation du sang

(30) Priorität: 18.03.2004 DE 202004004263 U
(43) Veröffentlichungstag der Anmeldung: 21.09.2005
(73) Patentinhaber: Med Tech Solutions GmbH, 90556 Seukendorf (DE)
(72) Erfinder: Weber, Waldfried, 90556 Seukendorf (DE)
(74) Vertreter: Tergau & Walkenhorst

(56) Entgegenhaltungen:
- WO-A-99/59645
- WO-A-03/026724
- US-A- 4 573 962
- US-A1- 2003 146 162
- US-A1- 2004 021 089
- US-B1- 6 491 656

## Beschreibung

Die Erfindung bezieht sich auf ein Bestrahlungsgerät für Blut, insbesondere zur Bestrahlung von Leukozyten, mit einem Bestrahlungsbehälter, durch den das Blut fließt, mit UVA-Bestrahlungslampen und einer Pumpe für die Bewegung des Blutes sowie einem Rezirkulationsbeutel für die Zwischenspeicherung des Blutes, wobei das Gerät Führungen zum Einschub eines für UVA - Strahlen durchlässigen Wagens in das Bestrahlungsfeld der Bestrahlungslampen besitzt, wobei ferner in den Wagen ein UVA - lichtdurchlässiger flacher Beutel einlegbar ist, der mit seinem Ausgang am Eingang des Rezirkulationsbeutels angeschlossen ist, wobei ein UVA-Sensor im Bestrahlungsraum zum Messen der realen Energieabgabe der Bestrahlungslampen vorgesehen und wobei ein Prozessor zur Steuerung der Funktionen des Gerätes vorhanden ist.

Das Bestrahlungsgerät ist gedacht für die Durchführung der extrakorporalen Photopherese, welche in der medizinischen Literatur seit etwa 1980 in zunehmendem und umfangreichem Maße beschrieben wurde und den Eingang auch in einer Reihe von Patenten gefunden hat. Seit einigen Jahren ist diese Methode in Europa eingeführt worden und wird beispielsweise mit einem Gerät durchgeführt, das unter dem Namen Therakos im Handel ist.

Die extrakorporale Photopherese wird bei Leukämie oder bei Abstoßreaktionen bei Transplantationen angewandt. In jüngerer Zeit ist aber auch ihre Anwendung bei einer Reihe von anderen Autoimmunerkrankungen beschrieben worden, wie z.B. die rheumatische Arthritis oder die fortschreitende systemische Sklerose. Auch bei dem Ausbruch des jugendlichen Diabetes melitus oder bei der Bekämpfung des Hepatitis C -Virus wird die Anwendung dieser Behandlungsmethode beschrieben.

Bei dem Verfahren der extrakorporalen Photopherese wird dem Patienten Blut entnommen und zunächst separiert in rote Blutkörperchen, Plasma und weiße Blutkörperchen (Leukozyten). Das mit Leukozyten angereicherte Blutplasma wird mit einer speziellen Kochsalzlösung (hämatosierter Kochsalzlösung) versehen und in den eigentlichen Bearbeitungskreislauf eingegeben. Durch Zugabe eines die Leukozyten für Licht sensibilisierenden Wirkstoffes, vorzugsweise 8Methoxy-Psoralen, wird das Blutplasma für die Bestrahlung vorbereitet. In einer nachgeordneten Bestrahlungsstation durchläuft das Blut einen Bestrahlungsbehälter zwischen mehreren UVA-Lampen und wird dort für etwa drei Stunden mit UVA-Licht der Wellenlänge von etwa 360nm bestrahlt. Beim Absterben von Zellen aufgrund der Bestrahlung entstehen Zerfallsprodukte, sogenannte Photo-Adducts, welche sich an andere Zellen anlagern und deren Zerfallsprozess beschleunigen. Auf diese Weise werden eine ganze Reihe von Zellen im Blutplasma durch den Zerfall an ihrem weiteren Wachstum gehindert. Durch diese Wirkung kommt es zu einer sogenannten Immunmodulation, wodurch es zu einer Verbesserung des Immunsystemes mit körpereigenen Zellen kommt. Nachdem das Blut bestrahlt worden war, wird es zusammen mit den vorab ge trennten roten Blutkörperchen und dem entnommenen restlichen Plasma dem Patienten wieder zugeführt. Diese Rückgabe des Blutes kann entweder in einem geschlossenen Kreislauf, an den der Patient unmittelbar angeschlossen ist, erfolgen, oder das bestrahlte Blutplasma kann zunächst zwischengespeichert werden und zu einem späteren, geeigneten Zeitpunkt zusammen mit den roten Blutkörperchen und dem restlichen Plasma an den Patienten zurückgegeben werden.

Das vorbeschriebene Verfahren ist im Detail in den US-Patenten 4 428 744, 4 683 889 und in der Internationalen Anmeldung WO97/36634 beschrieben. Dort ist eine Zentrifuge vorgesehen, welche das Blut vor der Rückgabe an den Patienten aufbereitet. Für die praktische Durchführung des Verfahrens ist das erwähnte Gerät Therakos auf dem Markt, welches den Plattenten in den geschlossenen Blutkreislauf einbezieht

Aus der US 2003/0146162 A1 ist bereits ein Gerät der vorbeschriebenen Art bekannt, bei dem ein beweglicher Wagen, in den ein mit dem zu untersuchenden Blut gefüllter Beutel eingelegt wird, in den Bestrahlungraum einschiebbar ist. Dort überwacht ein UVA - empfindlicher Sensor die Energiemenge die von den Lampen in den Bestrahlungsraum eingebracht wird. Ein Temperatursensor überwacht die Temperatur in dem Bestrahlungsraum, ein Lüfter kann die Temperatur konstant halten. Schließlich ist ein Prozessor vorgesehen, der die Sensoren steuert und die Eingabe und Speicherung der Blutdaten ermöglicht.

Aus der Wo 03/026724 A1 ist ferner bereits ein Blutbearbeitungsgerät bekannt, mit einem Bestrahlungsraum, in dem das in einem Beutel befindliche Blut mit Licht durchstrahlt wird. Die Besonderheit ist hier ein Lesegerät, mit welchem die Eigenschaften des Blutes, die an dem Beutel gespeichert sind, in das Gerät eingelesen werden können.

Aus der US 6 491 656 B1 ist weiterhin ein Blutbearbeitungsgerät bekannt, das einen Rezirkulationsbeutel für das Blut aufweist, der Beutel mäanderförmige Durchflußkanäle besitzt und ein Filter bei der Bestrahlung des Blutes verwendet. Nachteilig bei dieser Anordnung ist die Verwendung einer Zentrifuge.

Aus der WO 03/ 026 724 A1 ist schließlich ein Bestrahlungsgerät für Blut bekannt, mit einem Blutbeutel, der in das Bestrahlungsgerät eingelegt wird. Dieser Blutbeutel trägt an seinem Rahmen einen Chip, der die Daten des in ihm enthaltenen Blutes gespeichert hat. Dieser Chip kann von einem ortsfesten oder ortsbeweglichen Lese- und Schreibgerät ein- oder ausgelesen werden.

Die Erfindung hat es sich zum Ziel gesetzt, zur Durchführung der extracorporalen Photopherese ein Gerät anzugeben, bei welchem auf die Verwendung einer Zentrifuge zur Zwischenspeicherung des Blutes vor der Rückgabe des Blutes an den Patienten verzichtet werden kann, bei welchem ferner gewährleistet ist, dass nur für dieses Gerät zugelassene Bestrahlungsbeutel verwendet werden können.

Zur Lösung dieser Aufgabe schlägt die Erfindung vor, dass der Beutel mäanderförmige Durchlasskanäle besitzt und dass sein Zuleitungsschlauch über die Pumpe am Ausgang des Rezirkulationsbeutels angeschlossen ist und dass schließlich an dem Bestrahlungsgerät ein Chip-Karten-Lesegerät vorgesehen ist, welches Eingabemittel für die Daten und eine Anzeige zur Darstellung der Daten aufweist und welches mit dem Prozessor zusammenarbeitet und die Bestrahlung nur freigibt, wenn eine dem flachen Beutel zugeordnete Chip-Karte eingelegt ist.

Vorteilhaft ist die Erfindung dahingehend weitergebildet, dass ein Temperatursensor im Bestrahlungsraum zur Messung der Umgebungstemperatur angeordnet ist. Damit kann sichergestellt werden, dass das Blut in einem Temperaturbereich unterhalb 39,5°C behandelt wird.

Eine besonders vorteilhafte Weiterbildung der Erfindung sieht vor, dass ein Elektronikteil vorgesehen ist, welches einen Prozessor zur Steuerung der Funktionen des Gerätes enthält, wobei der Prozessor mit den Eingabemitteln, der Anzeige, sowie dem Temperatursensor und dem UVA - Sensor zusammenarbeitet und, abhängig von der Verarbeitung der Signale dieser Baugruppen, die Brennzeit der Bestrahlungslampen und die Schaltung der Pumpe steuert, sowie Abschaltsignale und etwaige Alarmsignale abgibt. Ein derart ausgerüstetes Bestrahlungsgerät ermöglicht die laufende Überwachung der Temperatur im Bestrahlungsraum und Regelung derselben, sowie über den UVA - Sensor die Berücksichtigung der mit zunehmender Lebensdauer geringer werdenden Abstrahlungsleistung der UVA - Lampen. Darüber hinaus ist gemäß weiterer Ausbildung an der digitalen Anzeige eine Menüführung darstellbar.

Weitere Merkmale der Erfindung sind aus den Unteransprüchen entnehmbar.

Im Folgenden soll die Erfindung anhand eines Ausführungsbeispieles unter Zugrundelegung der Zeichnungen noch näher erläutert werden.

Es zeigen:
- Figur 1: eine perspektivische Darstellung des Gerätes;
- Figur 2: eine perspektivische Darstellung des teilweise geöffneten Gerätes;
- Figur 3: eine schematisierte Darstellung des Blutkreislaufes für den Bestrahlungsvorgang;
- Figur 4: ein Blockschaltbild der elektronischen Steuerung.

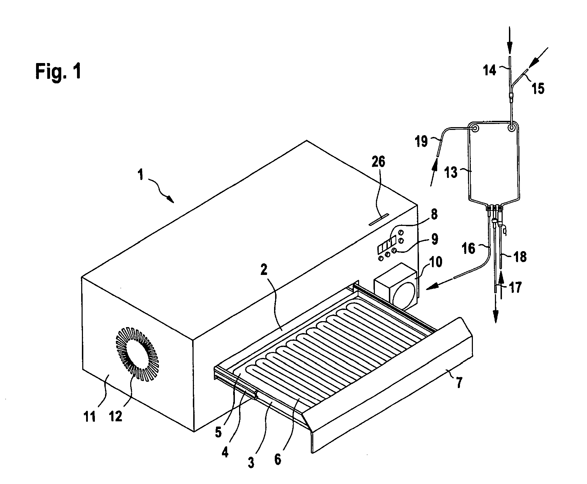

In Figur 1 ist an dem Bestrahlungsgerät 1 eine schlitzartige Öffnung 2 vorgesehen, in die ein verschiebbarer, in Gleitführungen 4 gelagerter Wagen 3, einschiebbar ist. In diesen verschiebbaren Wagen 3 ist ein flacher Beutel 5 einlegbar, welcher mäanderförmig angeordnete Durchflusskanäle 6 besitzt. Zur Abdeckung der Öffnung 2 im eingeschobenen Zustand besitzt der Wagen 3 eine Abdeckung 7.

An der Vorderseite des Gerätes 1 ist eine Digitalanzeige 8 vorgesehen, welche vorzugsweise als LED-Anzeige ausgebildet ist. Mit 9 sind mehrere Tasten bezeichnet, mittels welcher Daten und Start- bzw. Stoppsignale in das Gerät 1 eingegeben werden können. An der Vorderseite des Gerätes 1 ist ferner eine Pumpe 10 vorgesehen, welche zur Bewegung des Blutes im Blutkreislauf dient. An einer Seitenwand 11 sind Lüftungsschlitze 12 zur Kühlung des Bestrahlungsraumes im Gerät 1 vorgesehen. An den weiteren Seitenflächen des Gerätes 1 können noch eine oder zwei weitere Lüftungsöffnungen - nicht dargestellt - vorgesehen sein.

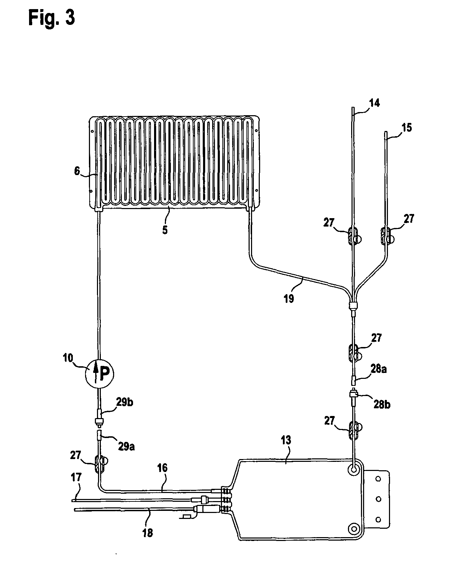

Außerhalb des Gerätes 1 befindet sich ein in der Zeichnung nur schematisch dargestellter Rezirkulationsbeutel 13, welcher zur Zwischenspeicherung des Blutes dient. Diesem werden über einen Eingangsschlauch 14 das bereits separierte Blut und ein mit Leukozyten angereichertes Blutplasma zugeführt. In diesen Schlauch 14 mündet ein weiterer Eingangsschlauch 15, über den heparinisierte Kochsalzlösung zugeführt wird. Der Ausgangsschlauch 16 ist mit der Pumpe 10 als Zuleitungsschlauch verbunden. Über einen Latex freien Ableitungsschlauch 17 wird das Blut nach dem Bestrahlungsvorgang an den Patienten zurückgegeben. Über einen weiteren Zuleitungsschlauch 18 wird dem Rezirkulationsbeutel 13 ein das Blut lichtsensibilisierender Wirkstoff in Form eines Photoaktivators, wie z.B. 8Methoxy-Psoralen zugeführt. Mit 19 ist schließlich der Ableitungsschlauch des Bestrahlungsbeutels 5 bezeich-net, über den das Blut nach der Bestrahlung wieder in den Rezirkulations-beutel 13 zurückgeführt wird. Die Anschlüsse der Schläuche zu und von der Pumpe sowie zu und von dem Bestrahlungsbeutel sind der Übersichtlich-keit halber in Figur 1 nicht dargestellt. Die Details hierzu sind aus der Figur 3 zu entnehmen.

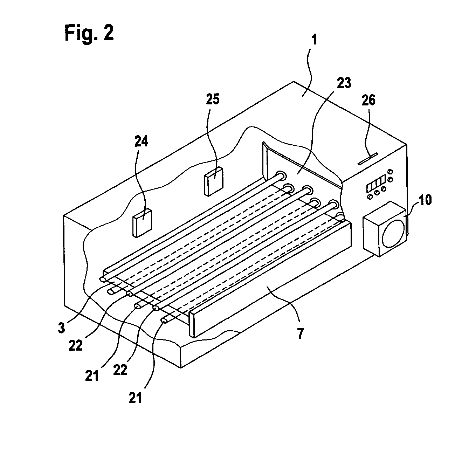

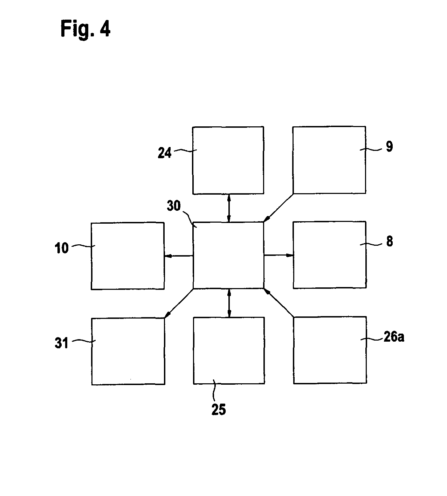

In Figur 2 ist das Gerät 1, teilweise aufgebrochen, dargestellt, so dass der Bestrahlungsraum sichtbar wird. Gleiche Teile sind mit gleichen Bezugszeichen wie in Figur 1 versehen. Der Wagen 3 besteht aus Acrylglas und ist in Figur 2 im eingeschobenen Zustand dargestellt. Man erkennt, dass unterhalb und oberhalb des Wagens 3 mehrere Leuchtstoffröhren 21 und 22 angeordnet sind, welche ein UVA - Licht in dem Wellenbereich von etwa 360 nm mit einer beiseitigen Toleranz von 25nm abgeben. Diese Leuchtstoffröhren 21,22 sind in seitlichen Wänden 23 gehaltert, von welchen die vordere Seitenwand der Übersichtlichkeit halber weggelassen ist. Mit 24 ist der Temperatursensor, mit 25 der UVA - Sensor bezeichnet. Wenngleich die beiden Sensoren 24 und 25 im Bestrahlungsraum oberhalb des Wagens 3 angeordnet sind, so ist es im Sinne der Erfindung natürlich ebenso möglich, diese beiden Sensoren 24,25 unterhalb des Wagens 3 oder sonst an geeigneter Stelle im Bestrahlungsraum anzuordnen. Der Temperatursensor 24 misst die Temperatur im Bestrahlungsraum. Steigt die Temperatur dort über 39,5°C, so gibt der Sensor 24 über die noch zu beschreibende Elektronik ein Alarmsignal ab, bei ansteigender Temperatur über 40°C schaltet er die Bestrahlungslampen ab. Die Elektronik kann dabei so ausgelegt sein, dass sie eine Regelfunktion auf die Bestrahlungslampen ausübt. Die Funktion des UVA-Sensors 25 soll im Zusammenhang mit der Beschreibung der Elektronik gemäß Figur 4 noch näher erläutert werden.

Als bevorzugte zusätzliche Ausstattung besitzt das Gerät 1 ein Chipkartenesegerät 26 mit einem Einschubschlitz. Jedem der Bestrahlungsbeutel 5 ist eine Chipkarte zugeordnet und wird mit diesem Beutel verkauft. Nur bei Einschub dieser Chipkarte wird das Gerät überhaupt gestartet. Auf diese Weise ist sichergestellt, dass nur die für dieses Gerät zugelassenen Bestrahlungsbeutel Verwendung finden, da andere Beutel mit möglicherweise anderen Wärme- oder Durchstrahl - Eigenschaften das Behandlungsergebnis des Blutes beeinträchtigen könnten. Auf diese Weise wird eine zusätzliche Sicherheit des Gerätes geschaffen.

Anhand der Figur 3 soll der Blutkreislauf mit Bestrahlung näher erläutert werden. Gleiche Bauteile sind mit gleichen Bezugszeichen wie in Figur 1 bezeichnet. Mit 27 sind mehrere Absperrventile, mit 28a, 28b und 29a, 29b sind zwei Trennelemente bezeichnet, durch welche der Blutkreislauf aufgetrennt werden und durch Zusammenfügen der Elemente 29a und 28b der Rezirkulationsbeutel 13 kurzgeschlossen werden kann.

Am Schlauch 14 wird das in einer Separationseinrichtung mit Leukozyten angereicherte Blutplasma gleichzeitig mit einer über den Schlauch 15 zugegebenen heparinisierten Kochsalzlösung dem Rezirkulationsbeutel 13 zugeführt. Dieser hat ein Volumen von bis zu 1000ml; bei Patienten, denen nur kleine Mengen Blut abgenommen werden können, kann vorteilhafterweise der gleiche Beute verwendet werden. Gleichzeitig mit dem Einlauf des Blutplasmas wird über den Schlauch 18 8Methoxy-Psoralen in den Beutel 13 gegeben, um das Blut für UVA - Licht zu sensibilisieren. Sobald die vorgesehene Menge Blut in den Rezirkulationsbeutel 13 eingelaufen ist, wird über die Starttaste am Gerät die Pumpe 10 eingeschaltet und pumpt nun das Blut langsam durch den Bestrahlungsbeutel 5 mit den mäanderförmigen Durchlaßkanälen 6. Gleichzeitig werden die Bestrahlungslampen 21 und 22 eingeschaltet und bestrahlen das durchfließende Blut mit der gewünschten Energiemenge von etwa 2 Joule/cm². Ein solcher Bestrahlungsvorgang dauert im allgemeinen bis zu 60 Minuten. Wenn die erforderliche Bestrahlungszeit abgelaufen ist, werden die Bestrahlungslampen 21, 22 ausgeschaltet und die Pumpe 10 von Vorwärtslauf in Rückwärtslauf umgeschaltet. Dies hat zur Folge, dass das Blut aus dem Blutkreislauf zurückgepumpt wird in den Rezirkulationsbeutel 13, wobei die dort befindliche Luft herausgedrückt wird. Nunmehr werden die Absperrventile 27 geschlossen und die Trennelemente 28a,28b und 29a,29b aufgetrennt und die Elemente 28b und 29a miteinander verbunden, so dass der Rezirkulationsbeutel 13 vom Bestrahlungskreislauf abgetrennt und sein Einlauf und Auslauf kurzgeschlossen sind. Die Behandlung des Blutes ist abgeschlossen und es kann zu einem gewünschten Zeitpunkt an den Patienten zurückgegeben werden. Die Einschaltung einer Zentrifuge ist dabei nicht mehr erforderlich. Durch diese Zwischenspeicherung des Blutes ist es möglich, diese Rückgabe in einem Zug oder auch in mehreren Teilen, zeitlich verzögert, vorzunehmen und die persönlichen gesundheitlichen Verhältnisse des Patienten zu berücksichtigen.

Das Bestrahlungsgerät 1 besitzt eine in Figur 4 dargestellte elektronische Steuereinheit mit einem Prozessor 30, der alle Funktionen des Gerätes 1 steuert. Er schaltet die Pumpe 10 und ein oder zwei Lüfter 31 ein und aus, er übernimmt die Signale der Tasten 9 und gibt Daten an die Anzeige 8 aus. Ferner arbeitet der Prozessor 30 mit dem Temperatursensor 24, dem UVA - Sensor 25 und dem Chipkartenlesegerät 26 zusammen.

Von besonderer Bedeutung für die Steuerung des Gerätes ist die Energiesteuerung für die UVA - Bestrahlungslampen. Um die notwendige Energiemenge für die Bestrahlung berechnen zu können, ist es erforderlich, dass vorab über die Tasten die vorgesehene Blutmenge sowie der Hämatokritwert eingegeben werden. Der Hämatokritwert wird in Prozent eingegeben und ist ein Maß für die Menge der roten Blutkörperchen. Je mehr von diesen vorhanden sind, desto größer ist die Absorption und desto geringer ist die durchdringende Energiemenge, die für die weißen Blutkörperchen übrig bleibt. Die zulässige durchschnittliche Energiezufuhrmenge ist in das Gerät von vornherein eingegeben und beträgt in etwa 2 Joule/cm². Aufgrund der eingegebenen Blutmenge und des Hämatokritwertes berechnet der Prozessor 30 die für die vorgesehene Bestrahlung notwendige Endenergiemenge. Abhängig von der Strahlungsleistung der UVA-Bestrahlungslampen 21 und 22 wird diese Energiemenge nach längerer oder kürzerer Zeit erreicht. Auf diese Weise wird die Alterung der UVA-Bestrahlungslampen 21,22 oder gar ein etwaiger Ausfall einer Lampe durch eine längere Bestrahlungsdauer ausgeglichen. Das Gerät kann dadurch sehr feinfühlig die Bestrahlung vornehmen und eine sehr gute und gleich bleibende Qualität des Ergebnisses erzielen. Darüber hinaus kann der Prozessor 30 durch Steuerung der Lüfter 31 die Temperatur im Bestrahlungsraum, die vom Temperatursensor 24 gemessen wird, konstant halten.

## Patentansprüche

1. Bestrahlungsgerät für Blut, insbesondere zur Bestrahlung von Leukozyten, mit einem Bestrahlungsbehälter, durch den das Blut fließt, mit UVA - Bestrahlungslampen (21,22) und einer Pumpe (10) für die Bewegung des Blutes, sowie einem Rezirkulationsbeutel (13) für die Zwischenspeicherung des Blutes, wobei das Gerät (1) Führungen (4) zum Einschub eines für UVA - Strahlen durchlässigen Wagens (3) in das Bestrahlungsfeld der Bestrahlungslampen (21,22) besitzt, wobei ferner in den Wagen ein UVA - lichtdurchlässiger flacher Beutel (5) einlegbar ist, dessen Ableitungsschlauch am Eingang des Rezirkulationsbeutels angeschlossen ist, wobei ein UVA - Sensor (25) im Bestrahlungsraum zum Messen der realen Energieabgabe der Bestrahlungslampen (21,22) vorgesehen und wobei ein Elektronikteil vorgesehen ist, welches einen Prozessor (30) zur Steuerung der Funktionen des Gerätes (1) enthält,
**dadurch gekennzeichnet,**
**dass** der Beutel (5) mäanderförmige Durchlaßkanäle (6) besitzt und dass sein Zuleitungsschlauch (16) über die Pumpe (10) am Ausgang des Rezirkulationsbeutels (13) angeschlossen ist und dass schließlich an dem Bestrahlungsgerät ein Chip-Karten-Lesegerät (26) vorgesehen ist, welches mit dem Prozessor (30) zusammenarbeitet und die Bestrahlung nur freigibt, wenn eine dem flachen Beutel (5) zugeordnete Chip-Karte eingelegt ist.

2. Bestrahlungsgerät nach Anspruch 1,
**dadurch gekennzeichnet,**
**dass** ein Temperatursensor (24) im Bestrahlungsraum zur Messung der Umgebungstemperatur angeordnet ist.

3. Bestrahlungsgerät nach den Ansprüchen 1 und 2,
**dadurch gekennzeichnet,**
**dass** der Prozessor (30) mit Eingabemitteln (9), einer Anzeige (8), sowie dem Temperatursensor (24) und dem UVA - Sensor (25) zusammenarbeitet und, abhängig von der Verarbeitung der Signale dieser Baugruppen, die Brennzeit der Bestrahlungslampen (21,22) und die Schaltung der Pumpe (10) steuert, sowie Abschaltsignale und etwaige Alarmsignale abgibt.

4. Bestrahlungsgerät nach einem der vorhergehenden Ansprüche,
**dadurch gekennzeichnet,**
**daß** eine oder mehrere Bestrahlungslampen (21,22) jeweils oberhalb und unterhalb des einschiebbaren Wagens (3) angeordnet sind.

5. Bestrahlungsgerät nach den Ansprüchen 1 und 3,
**dadurch gekennzeichnet,**
**daß** an dem Gerät (1) Tasten (9) zur Eingabe von Daten, wie Blutmenge, Hämatokritwert, und von Start- und Stopsignalen sowie weiterer etwa erforderlicher Daten vorgesehen sind.

6. Bestrahlungsgerät nach einem oder mehreren der vorhergehenden Ansprüche,
**dadurch gekennzeichnet,**
**daß** eine digitale Anzeige (8) vorgesehen ist, an welcher die eingegebenen Daten sowie Angaben zur Menüführung darstellbar sind.

7. Bestrahlungsgerät nach einem oder mehreren der vorhergehenden Ansprüche,
**dadurch gekennzeichnet,**
**daß** in dem Gerät (1) ein oder mehrere Lüfter (31) vorgesehen sind, die vorzugsweise von dem Prozessor (30) gesteuert sind.

8. Bestrahlungsgerät nach Anspruch 1,
**dadurch gekennzeichnet,**
**daß** der einschiebbare Wagen (3) einen Boden aus UVA-durchlässigem Acrylglas besitzt.

9. Bestrahlungsgerät nach den Ansprüchen 1 und 3,
**dadurch gekennzeichnet,**
**daß** im Prozessor (30) die durchschnittliche gewünschte Energiezufuhr pro Zeiteinheit und das gewünschte Endenergieniveau der Bestrahlung gespeichert sind und daß der Prozessor (30) ein Steuersignal an die UVA - Bestrahlungslampen (21,22) abgibt, das deren Einschaltdauer zeitlich variabel, abhängig von der eingegebenen, zu bestrahlenden Blutmenge dem eingegebenen Absorptionswert (Hämatokritwert) und der eingestrahlten UVA - Energiemenge bemißt.

## Claims

1. Blood irradiation device, in particular for the irradiation of leukocytes, having an irradiation container, through which the blood flows, having UVA irradiation lamps (21, 22) and a pump (10) for the movement of the blood and a recirculation bag (13) for the intermediate storage of the blood, wherein the device (1) possesses ducts (4) for the insertion of a carriage (3), which is permeable to UVA rays, into the irradiation field of the irradiation lamps (21, 22), wherein a flat, UVA-light-permeable bag (5) can also be inserted into the carriage, the outlet hose of which is connected to the entry of the recirculation bag, wherein a UVA sensor (25) is provided in the irradiation area for measuring the actual energy supply of the irradiation lamps (21, 22) and wherein an electronic part is provided, which contains a processor (30) for controlling the functions of the device (1),
**characterised in that**,
the bag (5) possesses meandering outlet channels (6), and **in that** its feed hose (16) is connected to the exit of the recirculation bag (13) via the pump (10), and **in that**, finally, a chip-card-reading device (26) is provided on the irradiation device, which operates in conjunction with the processor (30) and only releases the irradiation when a chip card assigned to the flat bag (5) is inserted.

2. Irradiation device according to claim 1,
**characterised in that**,
a temperature sensor (24) is arranged in the irradiation area for measuring the ambient temperature.

3. Irradiation device according to claims 1 and 2,
**characterised in that**,
the processor (30) operates in conjunction with input means (9), a display (8) and the temperature sensor (24) and the UVA sensor (25) and, depending on the processing of the signals of these components, controls the burning rate of the irradiation lamps (21, 22) and the switching of the pump (10), and emits switch-off and potential alarm signals.

4. Irradiation device according to one of the preceding claims,
**characterised in that**,
one or more irradiation lamps (21, 22) are arranged above and below the extendable carriage (3) respectively.

5. Irradiation device according to claims 1 and 3,
**characterised in that**,
buttons (9) for the input of data such as blood volume, haematocrit level and start and stop signals, as well as any further data required, are provided on the device (1).

6. Irradiation device according to one or more of the preceding claims,
**characterised in that**,
a digital display (8) is provided, on which the input data and menu navigation specifications can be displayed.

7. Irradiation device according to one or more of the preceding claims,
**characterised in that**,
one or more ventilators (31) are provided in the device (1), which are preferably controlled by the processor (30).

8. Irradiation device according to claim 1,
**characterised in that**,
the extendable carriage (3) possesses a floor made out of UVA-permeable acrylic glass.

9. Irradiation device according to claims 1 and 3,
**characterised in that**,
the average desired energy supply per unit of time and the desired final energy level of the irradiation are stored in the processor (30), and **in that** the processor (30) emits a control signal to the UVA irradiation lamps (21, 22), which variably measures their switch-on time, depending on the input blood volume to be irradiated, the input absorption level (haematocrit level) and the amount of irradiated UVA energy.

## Revendications

1. Appareil d'irradiation pour le sang, notamment pour l'irradiation de leucocytes, comprenant un caisson d'irradiation à travers lequel s'écoule le sang, comprenant également des lampes d'irradiation UVA (21, 22), et une pompe (10) pour assurer le mouvement du sang, ainsi qu'une poche de recirculation (13) pour l'emmagasinage temporaire du sang, appareil d'irradiation
dans lequel l'appareil (1) possède des guidages (4) pour insérer par coulissement un chariot (3) perméable aux rayons UVA, dans le champ d'irradiation des lampes d'irradiation (21, 22),
dans lequel il est possible de déposer dans le chariot, une poche plate (5) perméable à la lumière UVA et dont la tubulure de sortie d'évacuation est raccordée à l'entrée de la poche de recirculation,
dans lequel un capteur d'UVA (25) est prévu dans la chambre d'irradiation, pour mesurer l'émission réelle d'énergie des lampes d'irradiation (21, 22), et
dans lequel est prévue une partie électronique, qui englobe un processeur (30) pour la commande des fonctions de l'appareil (1),
**caractérisé en ce que** la poche (5) possède des canaux de passage (6) en forme de méandres, et **en ce que** sa tubulure d'alimentation (16) est raccordée, par l'intermédiaire de la pompe (10), à la sortie de la poche de recirculation (13), et **en ce qu'**il est finalement prévu sur l'appareil d'irradiation, un appareil de lecture de carte à puce (26), qui coopère avec le processeur (30) et ne libère ou valide l'irradiation que lorsqu'une carte à puce associée à la poche plate (5) est insérée.

2. Appareil d'irradiation selon la revendication 1,
**caractérisé en ce qu'**un capteur de température (24) est agencé dans la chambre d'irradiation, pour mesurer la température environnante.

3. Appareil d'irradiation selon les revendications 1 et 2,
**caractérisé en ce que** le processeur (30) coopère avec des moyens de saisie (9), un système de visualisation (8), ainsi qu'avec le capteur de température (24) et le capteur d'UVA (25), et, en fonction du traitement des signaux de ces composants, commande la durée d'allumage des lampes d'irradiation (21, 22) et la commutation de la pompe (10), et délivre également des signaux d'arrêt ou d'éventuels signaux d'alarme.

4. Appareil d'irradiation selon l'une des revendications précédentes,
**caractérisé en ce qu'**une ou plusieurs lampes d'irradiation (21, 22) sont agencées respectivement au-dessus et en-dessous du chariot coulissant insérable (3).

5. Appareil d'irradiation selon les revendications 1 et 3,
**caractérisé en ce que** sur l'appareil (1) sont prévues des touches (9) pour la saisie de données, comme la quantité de sang, la valeur d'hématocrite, et de signaux de démarrage et d'arrêt, ainsi que d'autres données éventuellement nécessaires.

6. Appareil d'irradiation selon l'une ou plusieurs des revendications précédentes,
**caractérisé en ce qu'**il est prévu un système de visualisation numérique (8) sur lequel peuvent être représentées les données saisies ainsi que des indications de gestion de menu.

7. Appareil d'irradiation selon l'une ou plusieurs des revendications précédentes,
**caractérisé en ce que** dans l'appareil (1) sont prévus un ou plusieurs ventilateurs (31), qui sont de préférence commandés par le processeur (30).

8. Appareil d'irradiation selon la revendication 1,
**caractérisé en ce que** le chariot coulissant insérable (3) possède un fond en un verre acrylique perméable aux UVA.

9. Appareil d'irradiation selon les revendications 1 et 3,
**caractérisé en ce que** dans le processeur (30) sont mémorisés l'amenée d'énergie moyenne souhaitée par unité de temps et le niveau final d'énergie souhaité de l'irradiation, et **en ce que** le processeur (30) délivre un signal de commande aux lampes d'irradiation UVA (21, 22), qui dimensionne leur durée de marche de manière variable dans le temps, en fonction de la quantité de sang à irradier ayant été saisie, de la valeur d'absorption (valeur d'hématocrite) ayant été saisie, et de la quantité d'énergie UVA rayonnée pour l'irradiation.
